# EUROPEAN PATENT APPLICATION

(11) **EP 4 011 389 A1**
(43) Date of publication of application: **15.06.2022**
(21) Application number: 20852111.2
(22) Date of filing: 07.08.2020
(51) Int. Cl.: A61K 39/39, A61K 35/12, A61K 35/15, A61K 38/20, A61K 39/00, A61K 39/395, A61K 45/00, A61P 31/00, A61P 35/00, A61P 37/04, A61P 43/00, C12N 5/0783, C12N 5/10, C12N 15/12

(54) **COMBINED USE OF ARTIFICIAL ADJUVANT VECTOR CELLS AND AN IMMUNOSTIMULANT**

(30) Priority: 09.08.2019 JP 2019147019; 18.05.2020 JP 2020086468
(71) Applicant: RIKEN, Wako-shi Saitama 351-0198 (JP)
(72) Inventor: FUJII Shinichiro, Wako-shi, Saitama 351-0198 (JP); SHIMIZU Kanako, Wako-shi, Saitama 351-0198 (JP)
(74) Representative: J A Kemp LLP
(86) International application number: PCT/JP2020/030402
(87) International publication number: WO 2021/029368

(57) **Abstract**

The present invention provides a composition for use in activating innate immunity, comprising an immunostimulant. The present invention also provides a combination use of an immunostimulant and an artificial adjuvant vector cell.

## Description

### Technical Field

The present invention relates to a composition for use in activating innate immunity, the composition comprising an immunostimulant (immune checkpoint inhibitor or immune activator) that acts on an immune checkpoint to stimulate immunity. The present invention also relates to combined use of an immunostimulant (immune checkpoint inhibitor or immune activator) and an artificial adjuvant vector cell.

### Background Art

Cells co-expressing CD1d and a target protein and pulsed with a CD1d ligand are called artificial adjuvant vector cells (aAVCs). Innate immunity and acquired immunity in response to the target protein can be induced in a subject who has received the cells (Patent Literatures 1 to 3 and Non-Patent Literature 1).

Among immunostimulants (e.g., an immune checkpoint inhibitor or an immune activator), the immune checkpoint inhibitor inhibits the functions of immune checkpoint molecules to weaken or disable the inhibitory effects on T cells. This can enhance immunocompetence, thereby eliciting a therapeutic effect on cancer or infectious disease. The immunostimulant is an immune-stimulating formulation. More specifically, the immune checkpoint inhibitor can weaken or release suppression of antigen-specific cytotoxic T lymphocytes, which suppression has been caused by immune checkpoint molecules. This can facilitate the antigen-specific cytotoxic T lymphocytes to kill cancer cells or pathogen-infected cells (Patent Literature 4). The immune activator can enhance the functions of antigen-specific cytotoxic T lymphocytes.

### Citation List

### Patent Literature

Patent Literature 1: WO 2007/097370
Patent Literature 2: WO 2010/061930
Patent Literature 3: WO 2013/018778
Patent Literature 4: WO 2004/004771

### Non Patent Literature

Non Patent Literature 1: Shimizu K. et al., 2007, J. Immunol., Vol. 178, pp2853-2861

### Summary of Invention

The present invention provides a composition for use in activating innate immunity, the composition comprising an immunostimulant (immune checkpoint inhibitor or immune activator). The present invention also provides combined use of an immune checkpoint inhibitor and an artificial adjuvant vector cell.

The present inventors have found that an immunostimulant (immune checkpoint inhibitor or immune activator) can increase an invariant natural killer T cell (iNKT cell), which are involved in innate immunity, and can increase antigen-specific cytotoxic T lymphocytes (CTLs, or CD8-positive T-lymphocytes). The present inventors have also found that use of an immune checkpoint inhibitor in combination with an artificial adjuvant vector cell (aAVC) can increase iNKT cells and an antigen-specific cytotoxic T lymphocyte (CTL, or CD8-positive T-lymphocyte) to an antigen expressed on the aAVC. The present invention is based on these findings.

The invention can provide the following industrially applicable items of the invention.
(1) A pharmaceutical composition comprising an artificial adjuvant vector cell, to be used in combination with an immunostimulant (immune checkpoint inhibitor or immune activator), wherein
   the artificial adjuvant vector cell expresses CD1d, are pulsed with a CD1d ligand, and expresses an antigen.
(2) The pharmaceutical composition according to item (1), wherein the artificial adjuvant vector cell has at least one of an exogenous nucleic acid encoding CD1d and an exogenous nucleic acid encoding a tumor antigen.
(3) The pharmaceutical composition according to item (1) or (2), wherein the CD1d ligand is α-galactosylceramide.
(4) The pharmaceutical composition according to any one of items (1) to (3), wherein the immunostimulant is at least one antibody selected from the group consisting of an anti-PD-1 antibody, an anti-Lag3 antibody, an anti-TIM-3 antibody, an anti-CTLA4 antibody (these are immune checkpoint inhibitors), and an anti-OX40 antibody, and an anti-GITR antibody (hereinabove, immunostimulants).
(5) The pharmaceutical composition according to any one of items (1) to (3), wherein the immunostimulant is an anti-OX40 antibody.
(6) The pharmaceutical composition according to any one of items (1) to (3), wherein the immunostimulant is an immune-activating cytokine.
(7) An immunostimulant that acts on an immune-related molecule to stimulate immunity, to be used in combination with an artificial adjuvant vector cell or a pharmaceutical composition containing the cell, wherein
   the artificial adjuvant vector cell expresses CD1d, are pulsed with a CD1d ligand, and expresses an antigen.
(8) A combination formulation comprising an artificial adjuvant vector cell and an immunostimulant (immune checkpoint inhibitor or immune activator) that acts on an immune checkpoint to stimulate immunity, wherein
   the artificial adjuvant vector cell expresses CD1d, are pulsed with a CD1d ligand, and expresses an antigen.
(9) A composition for use in activating innate immunity, comprising an immunostimulant (immune checkpoint inhibitor or immune activator) that acts on an immune checkpoint to stimulate immunity.

### Brief Description of Drawings

[Figure 1] Fig. 1 is a scheme illustrating combined use of an immunostimulant (immune checkpoint inhibitor or immune activator) (antibody: Ab) and an artificial adjuvant vector cell (aAVC) in Examples.
[Figure 2] Fig. 2 shows flow cytometry of antigen-specific cytotoxic T lymphocytes (CD8-positive T-lymphocytes or CLTs) of a mouse in which an indicated immune checkpoint inhibitor was administered in combination with aAVCs expressing, as an antigen, ovalbumin (OVA) in accordance with the scheme of Fig. 1.
[Figure 3] Fig. 3 is graphs indicating the number and percentage of antigen-specific CTLs obtained in Fig. 2.

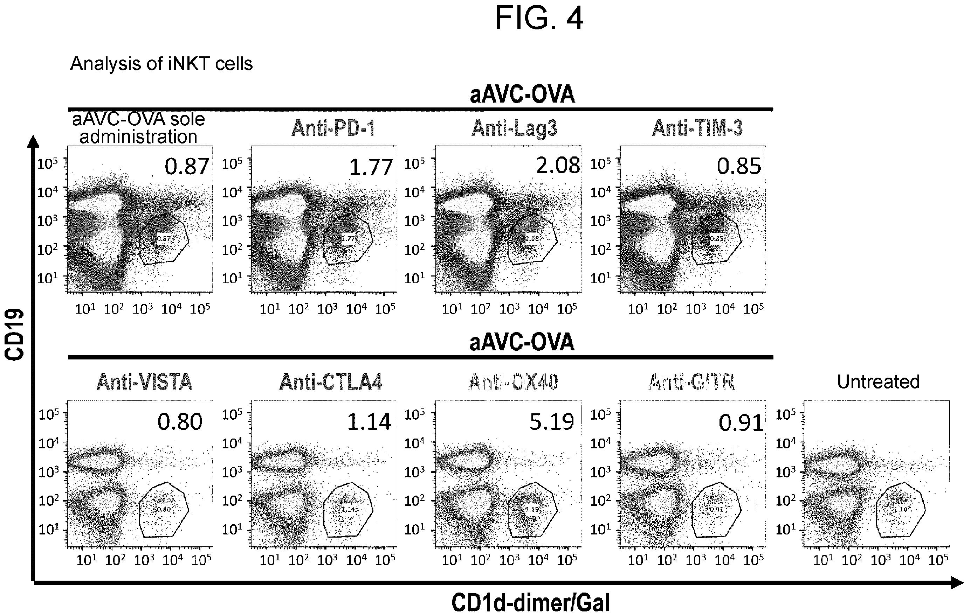

[Figure 4] Fig. 4 shows flow cytometry of invariant natural killer T-cells (iNKT cells) of a mouse in which an indicated immune checkpoint inhibitor was administered in combination with aAVCs expressing, as an antigen, ovalbumin (OVA) in accordance with the scheme of Fig. 1.
[Figure 5] Fig. 5 is graphs indicating the number and percentage of iNKT cells obtained in Fig. 4.
[Figure 6] Fig. 6 is a scheme illustrating combined use of an immunostimulant (IL-2) and an artificial adjuvant vector cell (aAVC) in Examples.
[Figure 7] Fig. 7 shows the percentage of antigen-specific T cells in the spleen of a mouse at day 14 in the scheme illustrated in Fig. 6.
[Figure 8] Figure 8 is a graphical representation of the results in Fig. 7.
[Figure 9] Fig. 9 is a scheme illustrating combined use of an immunostimulant (each indicated cytokine) and an artificial adjuvant vector cell (aAVC) in Examples.
[Figure 10] Fig. 10 shows the percentage (%) of antigen-specific T cells in the spleen of a mouse at day 14 in the scheme illustrated in Fig. 7.
[Figure 11] Fig. 11 is a graphical representation of the results in Fig. 10 (the number of antigen-specific T cells in spleen).
[Figure 12] Fig. 12 is a graphical representation of the results in Fig. 10 (the percentage (%) of antigen-specific T cells in spleen).
[Figure 13] Fig. 13 shows the percentage (%) of iNKT cells in the spleen of a mouse at day 14 in the scheme illustrated in Fig. 7.
[Figure 14] Fig. 14 shows the number of iNKT cells in the spleen of a mouse at day 14 in the scheme illustrated in Fig. 7.

### Detailed Disclosure of Invention

As used herein, the "subject" may be a mammal. Examples include a human or a non-human mammal.

As used herein, the term "treatment" is used to include and mean therapeutic treatment and prophylactic treatment. As used herein, the term "therapy" is used to include and mean delay or termination of the progress of a disease or condition as well as improvement or cure of a disease or condition. As used herein, the term "prophylaxis" is used to include and mean suppression of the onset of a disease or condition as well as a decrease in incidence rate thereof.

As used herein, the "artificial adjuvant vector cells" (aAVCs) mean CD1d pulsed cells expressing CD1d and an antigen (e.g., a molecule expressed on a target, such as a cancer antigen, a viral antigen, and a microbial antigen). The CD1d pulsed cells may be obtained by culturing CD1d-expressing cells with a CD1d ligand. After culturing, a CD1d ligand not attached to the cells can be removed by washing. CD1d is a protein of the CD1 family and an MHC class I-like glycoprotein. CD1d presents a glycolipid and can activate, for instance, iNKT cells. Examples of the CD1d include human CD1d such as a protein having the amino acid sequence deposited as GenBank Accession No: AAH27926.1. The CD1d ligand means a glycolipid that can bind to CD1d. Examples of the CD1d ligand include α-GalCer (α-galactosylceramide), α-C-GalCer (α-C-galactosylceramide), iGB3 (isoglobotrihexosylceramide), GD3 (ganglioside 3), GSL-1 (α-bound glucuronic acid), or GSL-1'SA (galacturonic acid), which can be each used as a CD1d ligand. As a CD1d ligand, for example, α-GalCer or α-C-GalCer may be preferably used.

Once aAVCs are administered to a mammal, iNKT cells are activated and dendritic cells are then matured. This can induce innate immunity and acquired immunity. If the aAVCs express a protein, acquired immunity in response to the protein can also be induced. Use of any of dendritic cells, cancer cells, or other cells (e.g., somatic cells) as the aAVCs can induce innate immunity and acquired immunity. Regardless of autologous or allogeneic aAVCs, innate immunity and acquired immunity can be induced in a subject. In the case of allogeneic aAVCs, innate immunity and acquired immunity are induced in the subject, and the aAVCs themselves may then be eliminated as a non-self by the immune system of the subject. If a target protein is expressed on aAVC, acquired immunity specific to the target protein can be induced in a subject who has received the aAVC. In the aAVC, the target protein is the full-length protein or a partial peptide. The aAVC expresses CD1d and is pulsed with a CD1d ligand. The aAVCs may have an exogenous gene that encodes CD1d and is operably linked to a regulatory sequence. The aAVC may have an exogenous gene that encodes a target protein and is operably linked to a regulatory sequence. The aAVC may express an endogenous target protein.

As used herein, the "cell" is a mammalian cell and may be, for instance, a human cell. Examples of the cells include dendritic cells or non-dendritic cells. Examples of the cells include tumor cells or non-tumor cells. Examples of the cells include cancer cells or non-cancer cells. Examples of the cells include immortalized cells or primary cells. Examples of the cells include somatic cells or other cells. The cells may be autologous or allogeneic. Examples of the cells include human cells or a human cell line such as a human embryonic kidney cell line (e.g., HEK293 cells).

As used herein, the "innate immunity" refers to a system for detecting and eliminating, by phagocytosis and pattern recognition, a cell(s) having an invaded foreign substance and/or an abnormality. Neutrophils, macrophages, natural killer cells (NK cells), natural killer T-cells (NKT cells), and dendritic cells are primarily responsible for innate immunity. By contrast, the "acquired immunity" refers to a system for effectively eliminating a re-entered foreign substance by memorizing the features of the foreign substance. Lymphocytes such as T cells (cytotoxic T lymphocytes (CTL) and helper T cells) and B cells are primarily responsible for acquired immunity. The acquired immunity is established after the innate immunity, so that time until a response is required. In the innate immunity, dendritic cells present in peripheral tissues phagocytose and take up a pathogen(s) to decompose them into peptides. Next, the dendritic cells migrate to a lymph node and the spleen and undergo maturation, and then each present an antigen peptide to a T cell that directs acquired immunity. Subsequently, acquired immunity is induced. Accordingly, killer T cells and B cells are activated in response to the antigen. Thus, an attack against the foreign substance begins. In this way, innate immunity induces acquired immunity via dendritic cells. Immature dendritic cells have no antigen-presenting potential and have strong phagocytosis activity. As the cells mature, the phagocytosis activity is weakened and the antigen presentation potential is enhanced. The cells also express high levels of adhesion and co-stimulatory molecules and acquire a strong T cell activation potential. The aAVCs are a vaccine system by which activation of innate immune lymphocytes such as invariant natural killer T-cells (iNKT cells) is induced, and maturation of dendritic cells may then be promoted to stimulate both innate immunity and acquired immunity. The aAVCs can activate iNKT cells via CD1d bound to an iNKT cell ligand (e.g., α-GarCer). Each aAVC itself is destroyed inside the body and incorporated into a dendritic cell. In addition, the aAVCs recruit killer T cells to, for instance, tumor tissues.

As used herein, the "antibody" means an immunoglobulin and refers to a protein structured by linking two heavy chains (H-chains) and two light chains (L-chains) stabilized by a pair of disulfide bonds. Each heavy chain consists of a heavy chain variable region VH, heavy chain constant regions CH1, CH2, and CH3, and a hinge region positioned between CH1 and CH2. Each light chain consists of a light chain variable region VL and a light chain constant region CL. Among them, a variable region fragment (Fv) consisting of VH and VL directly participates in the antigen binding and is a region giving the antibody diversity. An antigen-binding region consisting of VL, CL, VH, and CH1 is called an Fab region, and a region consisting of the hinge region, CH2, and CH3 is called an Fc region.

In the variable region, those that come into direct contact with an antigen are particularly variable and are called complementarity determining regions (CDRs). Regions that have relatively few mutations and are other than the CDRs are called framework regions (FRs). Three CDRs are present in the variable region of a heavy or light chain, called heavy chain CDR1-3 or light chain CDR1-3, respectively, in the order from the N-terminal side. As used herein, the term "anti-A antibody" means an antibody that can bind to A. The anti-A antibody may bind specifically to A. As used herein, the wording "bind specifically to A" means that the binding affinity for A is stronger than the binding affinity for proteins other than A.

The antibody may be a polyclonal or monoclonal antibody. In addition, the antibody may be of any isotype: IgG, IgM, IgA, IgD, or IgE. The antibody may be produced by immunizing a non-human animal (e.g., a mouse, a rat, a hamster, a guinea pig, a rabbit, a chicken). The antibody may be a recombinant antibody or a chimeric antibody, a humanized antibody, a fully humanized antibody, or the like. The chimeric antibody refers to an antibody obtained by linking antibody fragments derived from different species.

The "humanized antibody" means an antibody in which the amino acid sequences characteristic of a non-human antibody are used to replace those at the positions corresponding to human antibodies. Examples include an antibody having heavy chain CDR1-3 and light chain CDR1-3 of an antibody produced by immunizing a mouse or rat and having all other regions, including four framework regions (FRs) of each of the heavy chain or light chain derived from a human antibody. Such an antibody may be called a CDR graft antibody. The term "humanized antibody" may include a human chimeric antibody.

The "human chimeric antibody" is an antibody of non-human origin in which the constant regions of the non-human antibody are replaced by the constant regions of a human antibody. For human chimeric antibodies, from the viewpoint of enhancing ADCC activity, the subtype of a human antibody used for the constant regions can be, for example, IgG1.

As used herein, the "antigen-binding fragment" refers to an antibody fragment that has binding affinity to an antigen. Specific examples include, but are not limited to, an Fab consisting of VL, VH, CL, and CH1 regions; an F(ab')₂, in which the two Fabs are linked by a disulfide bond in the hinge region; an Fv consisting of VL and VH; an scFv, a single-chain antibody consisting of VL and VH linked by an artificial polypeptide linker; or a bispecific antibody (e.g., in a diabody form, an scDb form, a tandem scFv form, a leucine zipper form).

As used herein, the "immune checkpoint inhibitor" means an agent that stimulates immunity by disabling suppression of immune cells, which suppression has been caused by immune checkpoint molecules. As used herein, the "immune activator" means an agent that activates immune cells (e.g., an agent that stimulates T cells, especially an agent that stimulates CTLs, such as an agent that stimulates immune co-stimulatory molecules). The immune activator may be a target-selective agent. As used herein, the immune checkpoint inhibitor and the immune activator are collectively referred to as an "immunostimulant". Examples of the immune checkpoint molecule include programmed cell death-1 (PD-1), cytotoxic T-lymphocyte-associated protein 4 (CTLA-4), T-cell immunoglobulin domain and mucin domain-3 (TIM-3), lymphocyte activation gene 3 (LAG 3), or V-type immunoglobulin domain-containing suppressor of T-cell activation (VISTA). Their immune checkpoints are PD-1 axis immune checkpoint, CTLA-4 axis immune checkpoint, TIM-3 axis immune checkpoint, LAG-3 axis immune checkpoint, and VISTA axis immune checkpoint. The immune checkpoint inhibitors, for example, can bind to immune checkpoint molecules or their ligands and inhibit the functions of their immune checkpoints. For example, the PD-1 axis immune checkpoint can be inhibited by blocking the binding of PD-1 to PD-L1 or PD-L2. In addition, the CTLA-4 axis immune checkpoint can be inhibited by blocking the binding of CTLA-4 to CD80 or CD86. In addition, the TIM-3 axis immune checkpoint can be inhibited by blocking the binding of TIM-3 to galectin-9. In addition, the LAG-3 axis immune checkpoint can be inhibited by blocking the binding of LAG-3 to MHC class II molecules. In addition, the VISTA axis immune checkpoint can be inhibited by blocking the binding of VISTA to VSIG-3/IGSF11. The above makes it possible to inhibit at least one immune checkpoint selected from the group consisting of PD-1 axis immune checkpoint, CTLA-4 axis immune checkpoint, TIM-3 axis immune checkpoint, Lag-3 axis immune checkpoint, and VISTA axis immune checkpoint. Each antibody that can block the binding between the two proteins can bind to a receptor or a ligand. For example, the antibody that can inhibit the PD-1 axis immune checkpoint may be an antibody selected from the group consisting of anti-PD-1, anti-PD-Ll, and anti-PD-L2 antibodies (e.g., nivolumab, pembrolizumab, avelumab, atezolizumab, and durvalumab). In addition, the antibody that can inhibit the CTLA-4 axis immune checkpoint may be an antibody selected from the group consisting of anti-CDLA-4, anti-CD80, and anti-CD86 antibodies (e.g., ipilimumab, and tremelimumab). Further, the antibody that can inhibit the TIM-3 axis immune checkpoint may be an antibody selected from the group consisting of anti-TIM-3 and anti-galectin-9 antibodies (e.g., MGB453). Furthermore, the antibody that can inhibit VISTA axis immune checkpoint may be an antibody selected from the group consisting of anti-VISTA and anti-VSIG-3/IGSF11 antibodies (e.g., JNJ-61610588).

Next, examples of the immune co-stimulatory molecule include OX40 or a glucocorticoid-induced TNF-related protein (GITR). The immune co-stimulatory molecule such as GITR or OX40 may be stimulated by each agonistic antibody (i.e., an agonistic antibody capable of binding to GITR or an agonistic antibody capable of binding to OX40) or each agonist. In this case, killer T cells are activated and the functions of regulatory T cells are suppressed. In this way, the immune co-stimulatory molecules can be stimulated. This makes it possible to stimulate, for example, one or more immune co-stimulatory molecules selected from the group consisting of GITR and OX40. The immunostimulant against the GITR immune co-stimulatory molecule (e.g., an agonistic antibody or agonist against the GITR immune co-stimulatory molecule) may be an anti-GITR antibody or a GITR agonist (e.g., TRX518, MEDI1873). In addition, the immunomodulator against the OX40 immune co-stimulatory molecule (e.g., an agonistic antibody or agonist against the OX40 immune co-stimulatory molecule) may be an anti-OX40 antibody or an OX40 agonist (e.g., GSK3174998, MOXR0916, PF-04518600, MEDI0562). Another anti-OX40 agonistic antibody used may be any of the anti-OX40 agonistic antibodies described in WO2012027328A, WO2013028231A, WO2015153514A, or WO2015095423A.

The immune activator may be a pharmaceutical that can activate immunity. The immune-activating pharmaceutical may include an immune-activating cytokine such as a T cell-activating cytokine (e.g., a T cell-activating interleukin). Examples of the immune-activating cytokine include a cytokine such as IL-2. IL-2 is a cytokine classified in the group called Th1 cytokines, and is produced mainly by activated T cells. Human IL-2 protein, for example, can have the amino acid sequence registered as NBCI Accession No: NP_000577.2. Then, human IL-2 may be, for example, IL-2 with a sequence corresponding to the amino acid sequence registered as NBCI Accession No: NP_000577.2. Amino acids 1-20 of the amino acid sequence registered as NBCI Accession No: NP_000577.2 are considered to be a signal sequence. Thus, mature IL-2 has the amino acid sequence at positions 21 to 153. Accordingly, when IL-2 is expressed in a cell, a nucleic acid encoding IL-2 containing the signal sequence is introduced into the cell. When administered as a protein, IL-2 without the signaling sequence can be used.

Meanwhile, examples of the immune-activating pharmaceutical include a factor selected from the group consisting of IL-2, an IL-2/anti-IL-2 antibody complex, IL-7, IL-15, an IL-15/anti-IL15Rα antibody complex, IL-12, IL-18, IL-21, IL-23, and IL-27, which may be used in combination with aAVCs.

The IL-2/anti-IL-2 antibody complex is a complex of interleukin-2 (IL-2) and an anti-IL-2 antibody (i.e., an antibody that binds to IL-2). IL-2 is a factor that binds to an IL-2 receptor and triggers IL-2-mediated T cell proliferation. There are two types of anti-IL-2 antibodies: a regulatory T-cell (Treg)-inducing antibody that suppresses immunity and an immunostimulatory antibody that induces effector T cells. In the case of the former anti-IL-2 antibody (JES6-1 in the case of mice and 5344 in the case of humans), the antibody readily binds to cells that strongly express IL-2 receptor α-chain (CD25 (IL-2Rα)). Then, the complex acts on Treg. In the case of the latter anti-IL-2 antibody (e.g., S4B6 in the case of mice and MAB602, or TCB2 in the case of humans), the antibody readily binds to cells that express a high level of IL-2 receptor β-chain (CD122 (IL-2Rβ)), and the complex thereof reportedly acts on effector and memory T cells (e.g., CD4T and CD8T cells) and NK cells (PNAS 2010, 107: 11906; PNAS 2010, 107: 2171). In the invention, it is possible to use, for the formation of a complex with IL-2, an antibody that can bind more strongly to cells highly expressing the IL-2 receptor β-chain, such as an antibody that can bind more strongly to cells highly expressing the IL-2 receptor β-chain and enhance IL-2 functions (e.g., an antibody that acts on or induces cells selected from effector or memory T cells and NK cells). Here, the *in vivo* half-life of soluble IL-2 is short, only a few hours. The half-life of the IL-2/anti-IL-2 antibody complex, however, is reportedly prolonged. Thus, the functions of IL-2 can be enhanced by using a complex of IL-2 and an antibody that can bind to the IL-2 receptor β-chain. In particular, IL-2 and an anti-IL-2 antibody can be administered as a pre-formed complex. Thus, IL-2 can be administered in the form of a complex with an anti-IL-2 antibody. The IL-2 may be human IL-2, and the antibody may be an anti-human IL-2 antibody, a human chimeric antibody, a humanized antibody, or a human antibody. The human IL-2 is, for example, IL-2 having the amino acid sequence registered as GenBank Accession No: AAB46883.1 or human IL-2 having the corresponding amino acid sequence thereto. As used herein, the wording "having an amino acid sequence corresponding to amino acid sequence X" used means to include protein homologs (e.g., naturally occurring homologs) that retain the functions of a protein having the amino acid sequence X.

Interleukin-7 (IL-7) is a cytokine that plays an important role in T cell development and maintenance of naive and memory CD8-positive T-lymphocytes. The IL-7 may be human IL-7. Human IL-7 is, for example, IL-7 having the amino acid sequence registered as GenBank Accession No: AAH47698.1 or human IL-7 having the corresponding amino acid sequence thereto.

Interleukin-15 (IL-15) is a cytokine that, like IL-2, is involved in T cell activation and proliferation. The IL-15 may be human IL-15. Human IL-15 is, for example, IL-15 having the amino acid sequence registered as GenBank Accession No: CAA71044.1 or human IL-15 having the corresponding amino acid sequence thereto.

The IL-15/IL-15Rα complex will be described. IL-15 is a cytokine that can activate T cells, as described above. In addition, IL-15Ra is a receptor to which IL-15 binds. It has been known that IL-15, when bound to IL-15Ra and presented to effector and memory T cells (e.g., CD4 and CD8 T cells) and NK cells, stimulates these cells more potently (this phenomenon is called "trans-presentation") (Dubois S et al., Immunity, 2002, 17: 537). In addition, complexing IL-15 with IL-15Ra is said to prolong the half-life of IL-15 (Guo Y et al., Cytokine Growth Factor Rev, 2017, 38: 10). Thus, IL-15 as a complex with IL-15Ra can be administered to a subject. In this way, IL-15 in the form of a complex with IL-15Ra can be administered to a subject. The IL-15Ra may be human IL-15Ra. Examples include IL-15Ra having the amino acid sequence registered as GenBank Accession No: CAG33345.1 or human IL-15Ra having the corresponding amino acid sequence thereto.

Interleukin-12 (IL-12) is a heterodimer of IL-12A and IL-12B. IL-12 is believed to be involved in the activation and differentiation induction of T cells and NK cells. The IL-12 may be human IL-12 (i.e., a heterodimer of human IL-12 subunit α and human IL-12 subunit β). The human IL-12A may be a 35 kDa protein. Examples include IL-12A having the amino acid sequence registered as GenBank Accession No: AAI04985.1 or human IL-12A having the corresponding amino acid sequence thereto. Human IL-12B is, for example, IL-12B having the amino acid sequence registered as GenBank Accession No: EAW61576.1 or human IL-12B having the corresponding amino acid sequence thereto. IL-12 can be provided by complexing IL-12A with IL-12B.

Interleukin-18 (IL-18) is a cytokine that can induce interferon γ. IL-18 is believed to have functional similarities with IL-12. IL-18 exists intracellularly as a 24 kDa precursor, but is released extracellularly as a mature 18 kDa cytokine after cleaved by active caspase-1. In the invention, this mature form of IL-18 can be used as IL-18. The IL-18 may be human IL-18. Examples of the human IL-18 include human IL-18 having the amino acid sequence registered as UniProtKB/Swiss-Prot Accession No: Q14116.1 or human IL-18 having the corresponding amino acid sequence thereto.

Interleukin-21 (IL-21) is a type I cytokine with homology to IL-2 and IL-15. IL-21 is mainly produced by activated T cells. Examples of the IL-21 include human IL-21. The human IL-21 is, for example, IL-21 having the amino acid sequence registered as GenBank Accession No: EAX05226.1 or human IL-21 having the corresponding amino acid sequence thereto.

Interleukin-23 (IL-23) is a cytokine composed of IL-23 subunit α and the p40 subunit of IL-12. IL-23 is believed to promote the production and survival of a T-cell subset called TH17 cells. The IL-23 may be human IL-23. The human IL-23A may be, for example, IL-23 subunit α having the amino acid sequence at positions 20 to 189 of the amino acid sequence of human IL-23 subunit α precursor (amino acids 1 to 189) registered as NCBI Accession No: NP_057668.1 or having the corresponding amino acid sequence thereto. The IL-23 subunit α and the p40 subunit of IL-12 can be provided as a complex.

Interleukin-27 (IL-27) is a heterodimer of interleukin-30 (IL-30) and the Epstein-Barr virus-induced gene 3 (EBI3) subunit of interleukin-27. IL-27 has been implicated in the proliferation of antigen-specific naive CD4-positive T cells and in promoting their differentiation into the Th1 phenotype. The IL-30 may be human IL-30. Examples of the human IL-30 include human IL-30 having the amino acid sequence (amino acids 1 to 28 are considered to be a signal sequence) registered as UniProtKB/Swiss-Prot Accession No: Q8NEV9.2 or human IL-30 having the corresponding amino acid sequence thereto. In addition, the EBI3 subunit of IL-27 may be the EBI3 subunit of human IL-27. The EBI3 subunit of human IL-27 may be the human IL-27 EBI3 subunit having the amino acid sequence (amino acids 1 to 28 are considered to be a signal sequence) registered as UniProtKB/Swiss-Prot Accession No: Q8K3I6.1 or a human IL-27 EBI3 subunit having the corresponding amino acid sequence thereto.

In some embodiments, a signal peptide may be cleaved from any of cytokines selected from the group consisting of IL-2, IL-7, IL-15, IL-12, IL-18, IL-21, IL-23, and IL-27.

The present inventors have found that when an immunostimulant (immune checkpoint inhibitor or immune activator) is used in combination with aAVCs, the number and percentage of antigen-specific CTLs to an antigen expressed on the aAVCs are increased. The present inventors have also found that when an immune checkpoint inhibitor is used in combination with aAVCs, the number and percentage of iNKT cells are increased. The immune checkpoint inhibitor action is considered to inhibit its immune checkpoint(s) of the antigen-specific CTLs, thereby eliciting the intrinsic CTL action. According to the present inventors, the immune checkpoint inhibitor increased lymphocytes in the innate immune system and increased antigen-specific CTLs.

According to the invention, the immunostimulant (immune checkpoint inhibitor or immune activator) can activate innate immunity and can induce antigen-specific CTLs. Thus, the invention provides a composition for use in activating innate immunity and/or for use in inducing antigen-specific CTLs, comprising an immunostimulant (immune checkpoint inhibitor or immune activator). Here, the wording "inducing antigen-specific CTLs" means increasing the number and percentage of antigen-specific CTLs.

According to the invention, the immunostimulant (immune checkpoint inhibitor or immune activator) can enhance the ability of aAVCs to induce antigen-specific CLTs and iNKT cells. Thus, the invention provides:
(a) a pharmaceutical composition comprising an artificial adjuvant vector cell, to be used in combination with an immune checkpoint inhibitor, wherein the artificial adjuvant vector cell expresses CD1d, are pulsed with a CD1d ligand, and expresses a tumor antigen;
(b) a pharmaceutical composition comprising an immune checkpoint inhibitor, to be used in combination with an artificial adjuvant vector cell or a pharmaceutical composition containing the cells, wherein the artificial adjuvant vector cell expresses CD1d, are pulsed with a CD1d ligand, and expresses a tumor antigen;
(c) a combination formulation comprising an immunostimulant (immune checkpoint inhibitor or immune activator) and an artificial adjuvant vector cell, wherein
   the artificial adjuvant vector cell expresses CD1d, are pulsed with a CD1d ligand, and expresses a tumor antigen [the combination formulation may be a pharmaceutical composition comprising an immunostimulant (immune checkpoint inhibitor or immune activator) and an artificial adjuvant vector cell]; and
(d) a pharmaceutical composition for use in stimulating innate immunity, comprising an immune checkpoint inhibitor or a pharmaceutical composition for use in increasing antigen-specific CTL, comprising an immune checkpoint inhibitor
   (the above (a) to (d) are sometimes collectively referred to as a pharmaceutical in the invention).

The artificial adjuvant vector cells in the pharmaceutical composition in (a) or (b) or the combination formulation described in (c) can express endogenous CD1d. In addition, the artificial adjuvant vector cells may also express exogenous CD1d. The exogenous CD1d can be expressed on artificial adjuvant vector cells by introducing a nucleic acid encoding exogenous CD1d. If the nucleic acid is DNA, the DNA is operably linked to a promoter. The nucleic acid may be mRNA. When endogenous CD1d is highly expressed, it is not necessary to introduce exogenous CD1d into the artificial adjuvant vector cells.

The artificial adjuvant vector cells may express an antigen. Examples of the antigen include a tumor antigen or an antigen of an invading foreign substance.

The tumor antigen is an antigen that is more abundantly expressed in tumors than in normal cells. The tumor antigen may preferably be an antigen expressed in tumors, in terms of the number of surface molecules, at least 2, 3, 4, 5, 6, 7, 8, 9, 10, 20, 30, 40, 50, 60, 70, 80, 90, 100, 200, 300, 400, 500, 600, 700, 800, 900, or 1,000 times higher than in normal cells. Examples of the tumor antigen include an antigen in any of solid tumors, including epithelial and non-epithelial tumors, or tumors in hematopoietic tissues. Examples of the tumor antigen include, but are not limited to, MART-1, ZMelan-A, Mage-1, Mage-3, gpl00, tyrosinase, CEA, PSA, CA-125 erb-2, Muc-1, Muc-2, TAG-72, AES, FBP, C lectin, NY-ESO-1, galectin-4/NY-CO-27, Pec60, HER-2/erbB-2/neu, telomerase, G250, Hspl05, a ras oncogene point mutant, a p53 oncogene point mutant, or an oncoantigen such as an oncogenic embryonic antigen, or a neoantigen (see, for example, JP-2005-139118, JP-2004-147649, JP-2002-112780, JP-2004-222726). Examples of the tumor antigen in any of hematopoietic tissues (e.g., leukemia) include, but are not limited to, proteinase 3, WT-1, hTERT, PRAME, PML/RAR-a, DEK/CAN, cyclophilin, TEL-MAL1, BCR-ABL, OFA-iLRP, Survivin, idiotype, Sperm protein 17, SPAN-Xb, CT27, or MUC1.

Examples of the antigen of an invading foreign substance include an antigen of a pathogen. The antigen of a pathogen may be a pathogenic viral antigen, a pathogenic microbial antigen, or a pathogenic protozoan antigen. Examples of the pathogenic viral antigen include, but are not particularly limited to, GP-120, pl7, GP-160 (hereinabove, HIV), NP, HA (hereinabove, influenza virus), HBs Ag, HBV envelope protein, core protein, polymerase protein, NS3, NS5 (hereinabove, hepatitis virus), HSVdD (herpes simplex virus), EBNA1, 2, 3A, 3B or 3C, LMP1 or 2, BZLF1, BMLF1, BMRF1, BHRF1 (hereinabove, EB virus), Tax (HTLV-I), SARS-CoV spike protein (SARS virus), CMV pp50, CMV pp65, IE-1 (hereinabove, CMV), or E6 or E7 protein (hereinabove, HPV) (see, for example, JP-2004-222726). Examples of the pathogenic microbial antigen include an antigen expressed in any of pathogenic bacteria (e.g., *Chlamydia, Mycobacteria, Legionella*) or pathogenic yeast (e.g., *Aspergillus, Candida*). Examples of the pathogenic protozoan antigen include an antigen expressed in any of malaria or *Schistosoma.*

The artificial adjuvant vector cells may be isolated or purified cells. The artificial adjuvant vector cells may be a cell line. The artificial adjuvant vector cells may be human or non-human animal (e.g., non-human mammal, non-human primate) cells. Examples of the non-human animal include a monkey, a chimpanzee, a gorilla, an orangutan, a bonobo, a dog, a cat, a cow, a pig, sheep, a horse, a mouse, a rat, a guinea pig, a hamster, or a rabbit. The artificial adjuvant vector cells may be autologous, allogeneic, or heterologous cells. This is because they are eventually phagocytosed by dendritic cells.

The artificial adjuvant vector cells may be somatic cells. The somatic cell may be derived from, for instance, the stomach, small intestine (e.g., duodenum, jejunum, ileum, colon), colon, rectum, lung, spleen, kidney, liver, thymus, spleen, thyroid, adrenal gland, prostate, ovary, uterus, bone marrow, skin, or peripheral blood. The cells in the invention may also be of specific cell type in the above tissues or of cell type present in any of tissues other than the above tissues. Examples of such a cell type include epithelial cells, endothelial cells, epidermal cells, stromal cells, fibroblasts, adipocytes, mammary gland cells, mesangial cells, splenocytes, neurons, glial cells, immune cells (e.g., T cells, B cells, NK cells, NKT cells, macrophages, mast cells, neutrophils, basophils, eosinophils, monocytes), as well as progenitors or stem cells of the former cells.

The antigen expressed on the artificial adjuvant vector cell may be an antigen derived from the cell or an exogenously introduced antigen. In case where an antigen expressed on the artificial adjuvant vector cell may be an antigen derived from the cells, the artificial adjuvant vector cells can be created from, for example, a tumor cell or pathogen-infected cell. Examples of the pathogen-infected cells include virus-infected cells, microorganism-infected cells, or protozoa-infected cells.

The artificial adjuvant vector cell may be a non-transformant or a transformant. Transformation refers to artificial gene introduction. For instance, cells having DNA or mRNA introduced exogenously refers to transformants. In case where the artificial adjuvant vector cell may endogenously express CD1d and an antigen, the artificial adjuvant vector cell can be created without transformation.

The artificial adjuvant vector cells can be produced from an antigen-presenting cell (APC) or non-APC. Examples of the APC include a dendritic cell, a macrophage, a B cell, a Langerhans cell, or an activated T cell.

The artificial adjuvant vector cells can be created while pulsed with a CD1d ligand. The cells may be pulsed with a CD1d ligand such that CD1d-expressing cells are cultured in a culture medium in the presence of a CD1d ligand. In this way, the CD1d ligand can be presented to the cells that express a target antigen and CD1d. When the CD1d ligand is presented to the cells that express a target antigen and CD1d, immunity against the target antigen can be activated *in vivo.*

As the culture medium, it is possible to use, if appropriate, any culture medium that can be used for cell culture. Examples of the culture medium include MEM medium, DMEM medium, αMEM medium, Ham medium, RPMI 1640 medium, Fischer's medium, or a mixture thereof. The culture medium may contain an additional component(s) selected from the group consisting of, for example, serum (e.g., FCS), serum substitute (e.g., Knockout Serum Replacement (KSR)), fatty acids or lipids, amino acids, vitamins, growth factors, cytokines, antioxidants, 2-mercaptoethanol, pyruvate, buffers, and inorganic salts.

A nucleic acid encoding exogenous CD1d and/or a nucleic acid encoding an exogenous antigen can be introduced into cells using a gene expression vector(s). The nucleic acid encoding exogenous CD1d and the nucleic acid encoding an exogenous antigen may be included in one gene expression vector or may be included in separate gene expression vectors. In case where the nucleic acid encoding exogenous CD1d and the nucleic acid encoding an exogenous antigen may be included in one gene expression vector, the nucleic acids may be contained in a polycistronic mRNA so as to be transcribed as one mRNA or may be contained in monocistronic mRNAs so as to be transcribed as separate mRNAs. In the case of the two genes are contained in a polycistronic mRNA, an internal ribosome entry site (IRES) may be inserted between the respective genes. Each of the nucleic acid encoding exogenous CD1d or the nucleic acid encoding an exogenous antigen may be operably linked to a promoter suitable for transcription of mRNA in a cell. The promoter means the smallest sequence that is sufficient to induce transcription in a cell. Examples of the promoter include a viral promoter, a mammalian promoter, or a yeast promoter (e.g., mammalian CMV promoter, yeast alcohol oxidase promoter, phosphoglycerokinase promoter, lactose-inducible promoter, galactosidase promoter, adeno-associated virus promoter, baculovirus promoter, pox virus promoter, retrovirus promoter, adenovirus promoter, SV40 promoter, HMG (hydroxymethylglutaryl coenzyme A) promoter, TK (thymidine kinase) promoter, 7.5K or H5R poxvirus promoter, adenovirus type 2 MPC late promoter, α-antrypsine promoter, factor IX promoter, immunoglobulin promoter, CFTR surfactant promoter, albumin promoter, transferrin promoter). The nucleic acid encoding exogenous CD1d or the nucleic acid encoding an exogenous antigen may be mRNA or, for example, the above mRNA. The mRNA contains elements necessary for the translation of the encoded protein, and when introduced into the cell, it triggers the translation of the encoded protein. Gene transfer to cells can be performed by conventional procedures.

Among the immunostimulants in the invention, examples of the immune checkpoint inhibitor include PD-1 axis immune checkpoint inhibitor, CTLA-4 axis immune checkpoint inhibitor, TIM-3 axis immune checkpoint inhibitor, Lag-3 axis immune checkpoint inhibitor, or VISTA axis immune checkpoint inhibitor. Among the immunostimulants, examples of the immune activator include an immune activator for GITR or an immune activator for OX40 (each agonist or at least one antibody selected from each agonist antibody such as a GITR antibody or an OX40 antibody). In the invention, an OX40 antibody, which is an immune activator for OX40, is more preferable.

In the invention, aAVCs and an immunostimulant (immune checkpoint inhibitor or immune activator) may be administered simultaneously or separately. In the invention, aAVCs may be administered as a single dose or as multiple doses. In the invention, the immunostimulant (immune checkpoint inhibitor or immune activator) may be administered as a single dose or as multiple doses. In a certain embodiment of the invention, the aAVCs may be administered as a single dose, and the immunostimulant (immune checkpoint inhibitor or immune activator) may be administered as multiple doses.

In the invention, aAVCs and an immunostimulant (immune checkpoint inhibitor or immune activator) may be provided as one package or separate packages. If provided as one package, the aAVCs and the immune checkpoint inhibitor may be mixed in an identical pharmaceutical composition or may be contained in separate pharmaceutical compositions. In an embodiment of the invention, the aAVCs and the immune checkpoint inhibitor are separately provided in different packages. In an embodiment of the invention, the aAVCs and the immune checkpoint inhibitor are provided in different pharmaceutical compositions in one package. An embodiment of the invention may further include aAVCs and/or an immunostimulant (immune checkpoint inhibitor or immune activator) and a package insert containing instructions for combined use of the immunostimulant (immune checkpoint inhibitor or immune activator) and the aAVC.

In an embodiment of the invention, the aAVCs may be provided in a frozen state. In a certain embodiment of the invention, the immunostimulant (immune checkpoint inhibitor or immune activator) may be provided as an infusion or an injection. In a certain embodiment of the invention, the aAVCs may be provided in a frozen state and sterilized state with γ-radiation.

The invention also provides a pharmaceutical composition for use in stimulating innate immunity (in particular, iNKT cells), comprising an immunostimulant (immune checkpoint inhibitor or immune activator). Innate immunity (in particular, iNKT cells) may be stimulated to increase antigen-specific CTLs. Thus, the invention also provides a pharmaceutical composition for use in increasing antigen-specific CTLs, comprising an immunostimulant (immune checkpoint inhibitor or immune activator). According to the invention, the antigen-specific CTLs may be increased by stimulating innate immunity. In an embodiment of the invention, examples of the immune checkpoint inhibitor include at least one immune checkpoint inhibitor (e.g., an antibody) selected from the group consisting of PD-1 axis immune checkpoint inhibitor, CTLA-4 axis immune checkpoint inhibitor, TIM-3 axis immune checkpoint inhibitor, Lag-3 axis immune checkpoint inhibitor, and VISTA axis immune checkpoint inhibitor. In this embodiment, also included is an activator for at least one immune costimulatory molecule selected from the group consisting of GITR and OX40 (e.g., OX40L, an agonist, an agonistic antibody). Particularly preferred may be an activator for OX40 (e.g., OX40L, an agonist or agonistic antibody against OX40).

In a certain embodiment of the invention, a pharmaceutical composition for use in stimulating innate immunity (particularly iNKT cells) may be a pharmaceutical composition for use in proliferating iNKT cells. In a certain embodiment of the invention, a pharmaceutical composition for use in stimulating innate immunity (particularly iNKT cells) or a pharmaceutical composition for use in proliferating iNKT cells may comprise an immunostimulant selected from the group consisting of an anti-PD-1 antibody, an anti-Lag3 antibody, an anti-VISTA antibody, an anti-OX40 antibody, and an anti-CTLA4 antibody (e.g., an anti-OX40 antibody as an agonist antibody).

In a certain embodiment of the invention, a pharmaceutical composition for use in increasing antigen-specific CTLs may be an immunostimulant selected from the group consisting of an anti-PD-1 antibody, an anti-TIM3 antibody, an anti-CTLA4 antibody and an anti-OX40 antibody (e.g., an anti-OX40 antibody as an agonist antibody).

The OX40 agonistic antibody may be produced such that, for example, hybridoma producing antibodies capable of binding to OX40 are screened for affinity of the antibody produced by each hybridoma to OX40; then, the resulting OX40 antibodies are selected using an increase in luciferase activity as an indicator while using OX40-expressing cells (e.g., HEK293 cells) that express a reporter (e.g., luciferase) gene under control of an NF-κB response element. Such a test system can be purchased from, for instance, BPS Bioscience, Inc. (Catalog # 60482) .

The immunostimulant (immune checkpoint inhibitor or immune activator) in the invention may further include an excipient. The aAVCs in the invention may further include an excipient. The excipient included may be a suitable excipient for each. The immune checkpoint inhibitor in the invention may be administered by parenteral administration such as intravenous, intratumoral, subcutaneous, or intraperitoneal administration. The immune checkpoint inhibitor can be formulated in a dosage form suitable for each administration route. The aAVCs in the invention may be administered by parenteral administration such as intravenous, intratumoral, subcutaneous, or intraperitoneal administration. The aAVCs can be formulated in a dosage form suitable for each administration route.

A pharmaceutical in the invention may be used to treat cancer and/or infectious disease. Examples of the cancer include any of solid tumors (e.g., epithelial or non-epithelial tumors) or tumors in hematopoietic tissues. More specifically, examples of any of solid tumors that may be treated by a pharmaceutical in the invention include gastrointestinal cancer (e.g., stomach, colon, large intestine, rectum cancer), lung cancer (e.g., small cell, non-small cell cancer), or spleen cancer, kidney cancer, liver cancer, thymus cancer, spleen cancer, thyroid cancer, adrenal gland cancer, prostate cancer, bladder cancer, ovary cancer, uterine cancer (e.g., endometrial, cervical cancer), bone cancer, skin cancer, sarcoma (e.g., Kaposi's sarcoma), melanoma, blastoma (e.g., neuroblastoma), adenocarcinoma, squamous cell carcinoma, non-squamous cell carcinoma, brain tumor, or cancer due to recurrence or metastasis of each solid tumor. Examples of any of tumors in hematopoietic tissues that may be treated by a pharmaceutical in the invention include leukemia (e.g., acute myelogenous leukemia (AML), chronic myelogenous leukemia (CML), acute lymphocytic leukemia (ALL), chronic lymphocytic leukemia (CLL), adult T-cell leukemia-lymphoma (ATL), myelodysplastic syndromes (MDS)), lymphoma (e.g., T lymphoma, B lymphoma, Hodgkin lymphoma, non-Hodgkin's lymphoma), myeloma (multiple myeloma), or cancer due to recurrence or metastasis of each tumor. Examples of the infectious disease that can be treated by a pharmaceutical in the invention include infectious disease caused by each pathogen described above.

The invention provides a method of treating cancer or infectious disease in a subject in need thereof, comprising administering to the subject an immunostimulant (immune checkpoint inhibitor or immune activator) and administering to the subject an aAVC. The aAVC may express an antigen specific to the cancer or infectious disease (foreign substance causing the infectious disease) to be treated. According to the invention, a pharmaceutical in the invention can be administered. In a certain embodiment, the pharmaceutical in the invention may be administered until an increase in the number of cytotoxic T cells is found. In a certain embodiment of the invention, after the pharmaceutical in the invention is administered, a step of measuring the number of cytotoxic T cells may further be included. In a certain embodiment of the invention, after the pharmaceutical in the invention is administered, a step of detecting an increase in the number of cytotoxic T cells in an administration subject may be included. The cytotoxic T cells may have antigen specificity for an antigen expressed on the aAVCs. In a certain embodiment, a therapeutically effective dose is an amount at which therapeutic efficacy on cancer or infectious disease is elicited, but can be an amount at which cytotoxic T cells can be increased in the administration subject.

The invention provides use of an immunostimulant (immune checkpoint inhibitor or immune activator) and/or aAVCs in the manufacture of a pharmaceutical in the invention. According to the invention, there is provided an immune checkpoint inhibitor and/or aAVCs for use in a method of treatment in the invention.

### Examples

### Example 1: Use of Immune Checkpoint Inhibitor in Combination with Artificial Adjuvant Vector Cells (aAVC)

In this Example, how combined use of an immunostimulant (immune checkpoint inhibitor or immune activator) and an aAVC was effective was examined.

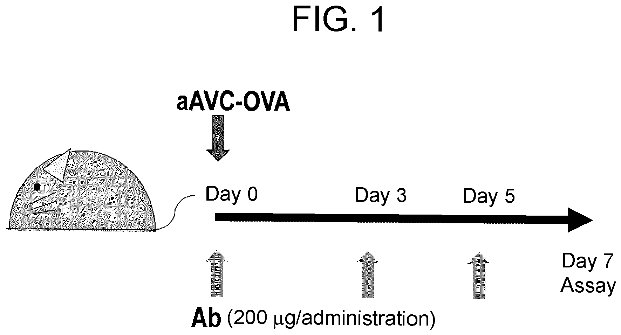

First, an aAVC and an immune checkpoint inhibitor were combined and administered to mice (C57BL/6 mice, 6-week-old, n = 2). The dosing scheme was as illustrated in Fig. 1. Specifically, an aAVC expressing ovalbumin (OVA) was administered on day 0; the immune checkpoint inhibitor (antibody) was administered at 200 µg/dose on days 0, 3, and 5; and on day 7, the number and percentage of antigen-specific CD8-positive T cells and the number and percentage of iNKT cells were checked.

The aAVC-OVA was obtained by expressing a gene encoding CD1d and a gene encoding OVA as an antigen in NIH3T3 cells, and then adding 0.5 mg/mL of α-galactosylceramide (α-GalCer) to the culture medium. The administration was performed at a dose of 5 × 10⁵ cells/animal.

Examples of the immunomodulator used include an immune checkpoint inhibitor such as an anti-PD-1 antibody (product number: BE0146; manufacturer: BioXcell), an anti-Lag3 antibody (product number: BE0174; manufacturer: BioXcell), an anti-TIM-3 antibody (product number: B8.2C12; manufacturer: BioXcell), an anti-VISTA antibody (product number: BE0310; manufacturer: BioXcell), or an anti-CTLA4 antibody (product number: BE0164; manufacturer: BioXcell). The immunostimulant used was an anti-OX40 antibody (product number: BE0031; manufacturer: BioXcell) or an anti-GITR antibody (product number: BE0063; manufacturer: BioXcell). Each dosing was performed at a dose of 200 µg/animal/administration.

The aAVC-OVA was administered intravenously, and the immunomodulator was administered intraperitoneally.

The spleen of each mouse was collected on day 7 after aAVC-OVA administration. Then, antigen-specific CD8-positive T cells (cytotoxic T lymphocytes) and invariant natural killer T cells (iNKT cells) were analyzed. For the CD8-positive T cells, splenocytes were isolated from the spleen, stained with CD3, CD8, CD44 antibodies and an OVA-tetramer, and analyzed using a FACS (manufacturer BD, product name Canto II). The splenocytes were gated with CD3- and CD8-positive cells to give CD8 T cells, which were plotted in terms of CD44 and OVA-tetramer. While the CD44-positive and OVA-tet-positive cells were defined as OVA-specific CD8-positive T-lymphocytes, the number and percentage of the lymphocytes were measured. This percentage is the percentage (%) of OVA-specific CD8-positive T-lymphocytes in the CD8 T cells.

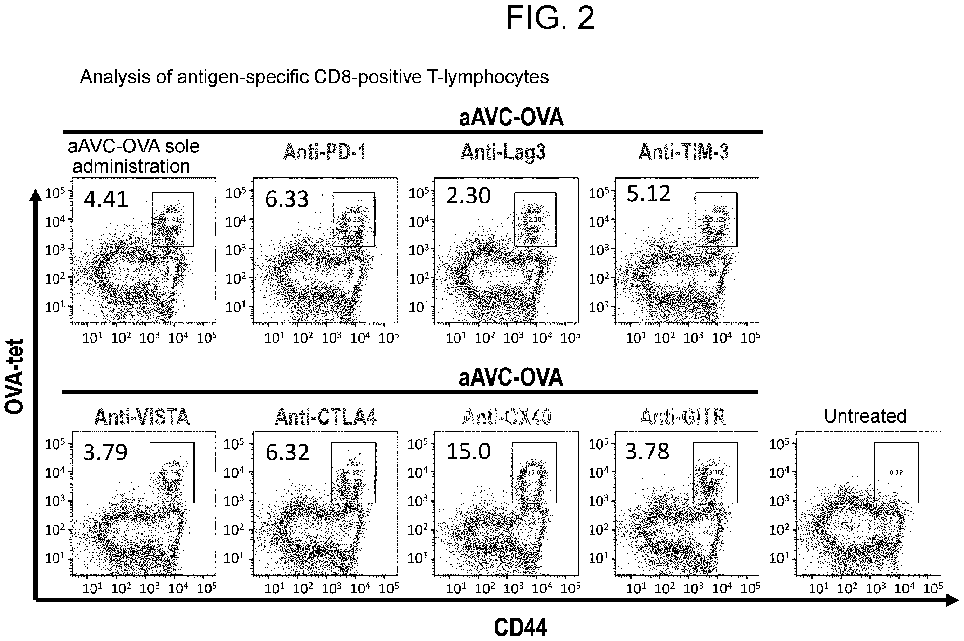

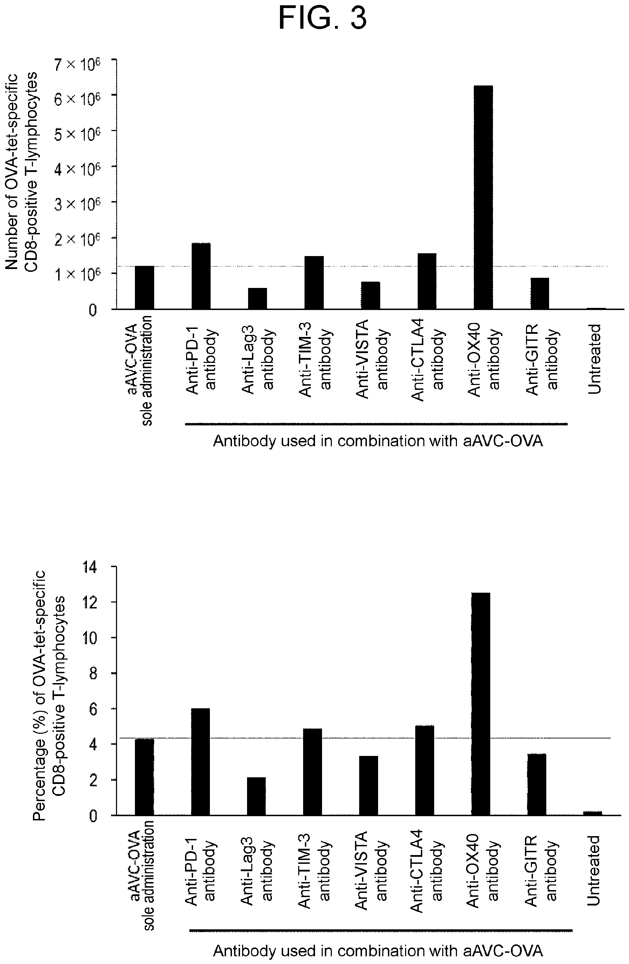

The results are shown in Fig. 2. As shown in Fig. 2, the percentage of OVA-specific CD8-positive T-lymphocytes in some of the immunostimulant combined use groups was found to be higher than that of the aAVC-OVA sole administration group. Specifically, the percentage of OVA-specific CD8-positive T-lymphocytes was increased in the combined use group with the anti-PD-1, anti-TIM-3, anti-CTLA4, or anti-OX40 antibody. In particular, there was a marked increase in the percentage of OVA-specific CD8-positive T-lymphocytes in the combined use group with the anti-OX40 antibody. The number and percentage of OVA-specific CD8-positive T-lymphocytes are shown graphically in Fig. 3. As shown in Fig. 3, the number and percentage of OVA-specific CD8-positive T-lymphocytes were increased in the combined use group with the anti-PD-1, anti-TIM-3, anti-CTLA4, or anti-OX40 antibody. In particular, there was a marked increase in the number and percentage of OVA-specific CD8-positive T-lymphocytes in the anti-OX40 antibody combined use group.

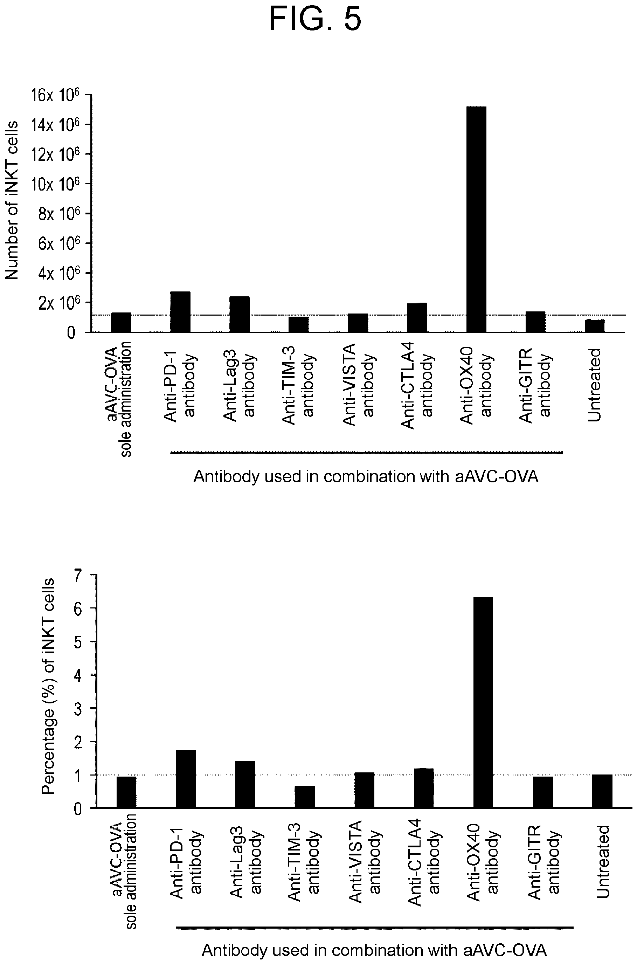

Further, the number and percentage of iNKT cells in the splenocytes were also analyzed. Specifically, each spleen was isolated 14 days after cell administration. The resulting splenocytes were stained with CD1d-tetramer/Gal-APC, which was an α-GalCer-preloaded CD1d tetramer labeled with allophycocyanin (APC), and CD3-PerCP/Cy5^{®}, which was a CD3 labeled with PerCP/Cy5^{®}. CD3⁺ and CD1d-tetramer/Gal⁺ cells were counted as iNKT cells. Next, the frequency of iNKT cells in the spleen was calculated. Further, the number of iNKT cells was calculated by multiplying the total number of cells in the spleen of each immunized mouse by the above frequency. As a result, as shown in Fig. 5, the number and percentage of iNKT cells were increased in the combined use group with the anti-PD-1, anti-Lag3, anti-VISTA, anti-OX40, or anti-CTLA4 antibody. In particular, the number and percentage of iNKT cells were increased in the anti-OX40 antibody combined use group.

Here, iNKT cells induce the activation of natural killer T cells (NK cells) and dendritic cells, which are responsible for innate immunity, as well as B cells and T cells, which are responsible for acquired immune responses. The aAVCs used in combination with an immune checkpoint inhibitor were demonstrated to enhance the potential to elicit innate immunity and acquired immunity.

Each immune checkpoint inhibitor enhances T-cell immunity by removing the suppression of T cells (cytotoxic T lymphocytes; CTLs), which are responsible for acquired immunity. The immune activator used in this study enhances T-cell immunity by directly stimulating the immune system. The present results indicate that the combined use of an immunostimulant and an aAVC enhances the stimulating action on innate immunity. It is impossible to simply assume that the aAVCs induced innate immunity and the immune checkpoint inhibitor enhanced acquired immunity as a result of which the additive effect was exerted.

The present results have revealed that the immunostimulant enhances the potential of aAVC to elicit innate immunity and simply release the suppression of cytotoxic T lymphocytes, and also increase the number of cytotoxic T lymphocytes, thereby providing a protocol for implementing more effective cancer immunotherapy.

Of course, each immune checkpoint inhibitor should enhance the abilities of aAVCs to stimulate innate immunity and induce antigen-specific immunity. Besides, the immunostimulant should also enhance an aAVC-mediated therapeutic effect on cancer or infectious disease.

### Example 2: Use of Immune Activator in Combination with aAVCs

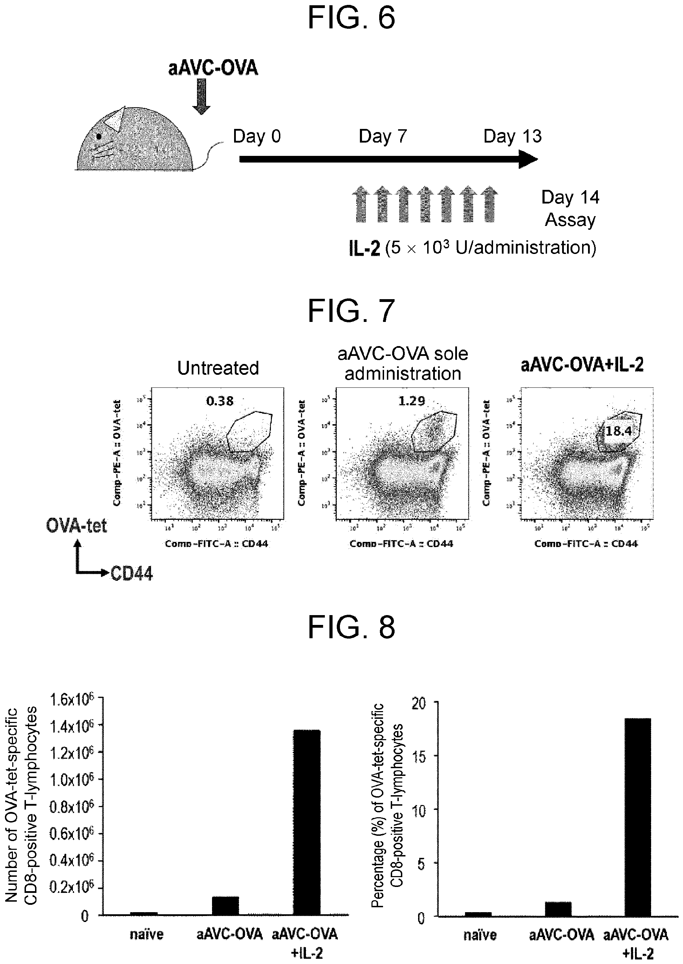

Next, the effect of the combination of an immune activator and aAVCs was checked by the scheme of Fig. 6, in which the aAVCs and a cytokine were used in combination. Like in Fig. 1, aAVC-OVA was administered to mice. IL-2 at 5 × 10³ U/dose was given daily for 7 days starting on day 7 after the administration. On day 14, the mouse spleen of each mouse was collected. The splenocytes were gated with CD3- and CD8-positive cells to give CD8 T cells, which were plotted in terms of CD44 and OVA-tetramer. While the CD44-positive and OVA-tet-positive cells were defined as OVA-specific CD8-positive T-lymphocytes, the number and percentage of the lymphocytes were measured. This percentage is the percentage (%) of OVA-specific CD8-positive T-lymphocytes in the CD8 T cells. The aAVC-OVA was prepared as in Example 1. The aAVC-OVA was administered at a dose of 5 × 10⁵ cells/animal as in Example 1.

The results are shown in Fig. 7 (flow cytometry results) and Fig. 8 (the number (left) and percentage (right) of antigen-specific CD8-positive T-lymphocytes). As shown in Figs. 7 and 8, the number and percentage of antigen-specific CD8-positive T-lymphocytes were significantly higher in the aAVC-OVA and IL-2 combined use group than in the case of aAVC-OVA sole administration.

This has demonstrated that the immune activator is useful when used in combination with aAVCs.

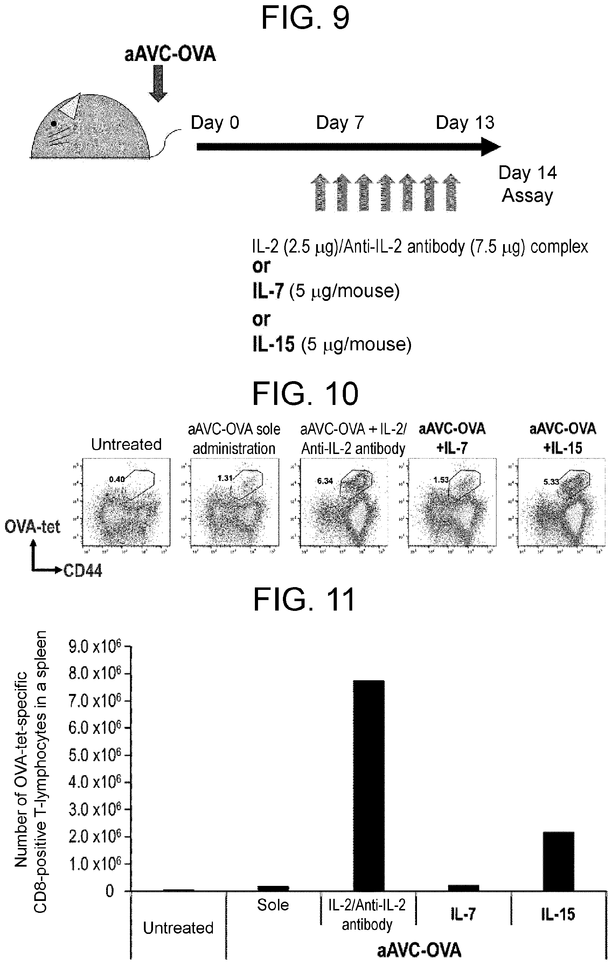

Further, the effect of the combination of an immune activator (i.e., an IL-2/anti-IL-2 antibody complex, IL-7, or IL-15) and aAVCs was checked by the scheme of Fig. 9, in which the aAVCs and each cytokine were used in combination. Like in Fig. 1, aAVC-OVA was administered to mice. Starting on day 7, each cytokine was administered daily for 7 days at each dose designated per mouse. On day 14, the mouse spleen of each mouse was collected. The splenocytes were gated with CD3- and CD8-positive cells to give CD8 T cells, which were plotted in terms of CD44 and OVA-tetramer. While the CD44-positive and OVA-tet-positive cells were defined as OVA-specific CD8-positive T-lymphocytes, the number and percentage of the lymphocytes were measured. This percentage is the percentage (%) of OVA-specific CD8-positive T-lymphocytes in the CD8 T cells. The aAVC-OVA was prepared as in Example 1. The aAVC-OVA was administered at a dose of 5 × 10⁵ cells/animal as in Example 1.

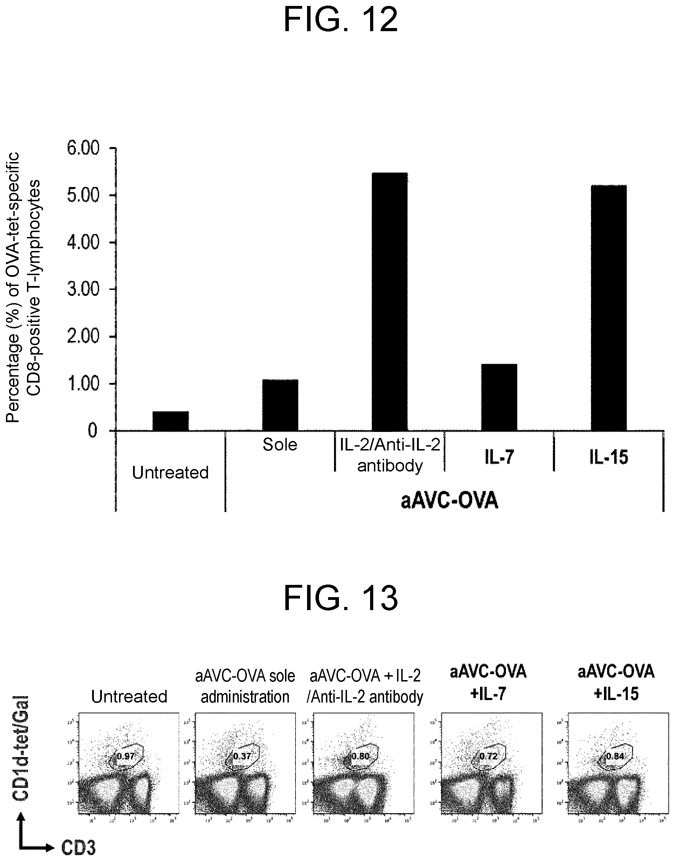

The results are shown in Fig. 10 (flow cytometry results), Fig. 11 (the number of antigen-specific CD8-positive T-lymphocytes in the spleen), and Fig. 12 (the percentage of antigen-specific CD8-positive T-lymphocytes). As shown in Figs. 10 to 12, the number and percentage of antigen-specific CD8-positive T cells were higher in the case of use in combination with any of the cytokines than in the case of aAVC sole administration. The increase was more pronounced when either the IL-2/anti-IL-2 antibody complex or IL-15 was administered.

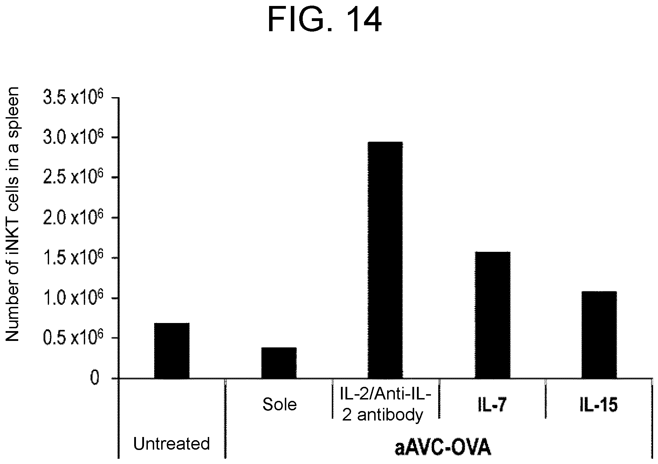

Further, as described in Example 1, the number and percentage of iNKT cells in the splenocytes were also analyzed. The results are shown in Fig. 13 (flow cytometry results) and Fig. 14 (the number of iNKT cells in the spleen). As shown in Figs. 13 and 14, the number of iNKT cells in the spleen was higher in the case of use in combination with any of the cytokines than in the case of aAVC sole administration. Thus, each administered immune activator was able to enhance the potential of the aAVCs to elicit innate immunity.

## Claims

1. A pharmaceutical composition comprising an artificial adjuvant vector cell, to be used in combination with an immunostimulant, wherein
the artificial adjuvant vector cell expresses CD1d, are pulsed with a CD1d ligand, and expresses an antigen.

2. The pharmaceutical composition according to claim 1, wherein the artificial adjuvant vector cell has at least one of an exogenous nucleic acid encoding CD1d and an exogenous nucleic acid encoding a tumor antigen.

3. The pharmaceutical composition according to claim 1 or 2, wherein the CD1d ligand is α-galactosylceramide.

4. The pharmaceutical composition according to any one of claims 1 to 3, wherein the immunostimulant is at least one antibody selected from the group consisting of an anti-PD-1 antibody, an anti-Lag3 antibody, an anti-TIM-3 antibody, an anti-CTLA4 antibody, and an anti-OX40 antibody.

5. The pharmaceutical composition according to any one of claims 1 to 3, wherein the immunostimulant is an anti-OX40 antibody.

6. The pharmaceutical composition according to any one of claims 1 to 3, wherein the immunostimulant is an immune-activating cytokine.

7. An immune checkpoint inhibitor, to be used in combination with an artificial adjuvant vector cell, or a pharmaceutical composition containing the cell, wherein
the artificial adjuvant vector cell expresses CD1d, are pulsed with a CD1d ligand, and express an antigen.

8. A combination formulation comprising an immunostimulant that acts on an immune checkpoint to stimulate immunity and an artificial adjuvant vector cell, wherein
the artificial adjuvant vector cell expresses CD1d, are pulsed with a CD1d ligand, and expresses an antigen.

9. A composition for use in activating innate immunity, comprising an immunostimulant that acts on an immune checkpoint to stimulate immunity.
